# EUROPEAN PATENT APPLICATION

(11) **EP 3 666 197 A1**
(43) Date of publication of application: **17.06.2020**
(21) Application number: 17921135.4
(22) Date of filing: 07.08.2017
(51) Int. Cl.: A61B 17/00

(54) **ENDOSCOPE OPERATING SHEATH FOR UROLOGICAL SURGERY**

(71) Applicant: Wuhan Youcare Technology Co., Ltd., Wuhan, Hubei 430223 (CN)
(72) Inventor: LONG, Gang, Wuhan Hubei 430223 (CN); LI, Jinping, Wuhan Hubei 430223 (CN); MAO, Yeyun, Wuhan Hubei 430223 (CN); PREMINGER, Glenn, Michael, Wuhan Hubei 430223 (CN); YE, Zhangqun, Wuhan Hubei 430223 (CN); LI, Jianxing, Wuhan Hubei 430223 (CN); YU, Xiao, Wuhan Hubei 430223 (CN); ZHANG, Junhui, Wuhan Hubei 430223 (CN); XIAO, He, Wuhan Hubei 430223 (CN); LI, Wencheng, Wuhan Hubei 430223 (CN); HU, Xuecheng, Wuhan Hubei 430223 (CN); WU, Yaohui, Wuhan Hubei 430223 (CN)
(74) Representative: Zeuner Summerer Stütz
(86) International application number: PCT/CN2017/096214
(87) International publication number: WO 2019/028591

(57) **Abstract**

An endoscope operating endoscopy sheath (2) for urological surgery, comprising a core rod (3) that can be disposed in the endoscope operating endoscopy sheath (2) and axially slides relative to the endoscope operating endoscopy sheath (2). The core rod (3) is internally provided with a guide wire passage (5), a water injection passage (6), and an imaging passage (7) that extend from the rear end to the front end of the core rod (3); an endoscope endoscopy handle (1) is provided with a guide wire connector (8), a water injection connector (9), and an endoscope connector (10) for respectively guiding a guide wire, water, and an endoscope into the guide wire passage (5), the water injection passage (6), and the imaging passage (7). The endoscope operating endoscopy sheath (2) and the core rod (3) enter a human body along the guide wire; the core rod (3) is drawn out after reaching a lesion site; then a flexible cystoscope is put into the endoscope operating endoscopy sheath (2); the endoscope operating endoscopy sheath (2) and the flexible cystoscope are operated to observe the lesion site and cooperate with surgical instruments for performing surgery. The method is simple and easy to implement; the endoscope operating endoscopy sheath can be widely applied to renal pelvis and renal caliceal calculi surgery, and may serve as a ureterectasia visualization endoscopy sheath, and is applicable to, but not limited to, cardiovascular intervention, gynecological surgery, thyroid gland and mammary gland surgery, neurosurgery and orthopedic surgery, and other unmentioned related clinical medical fields.

## Description

### TECHNICAL FIELD

This application invention relates to an endoscopy, and specially discloses an endoscope operating sheath for urological surgery.

### BACKGROUND

Endoscope is a high-end medical device that integrates materials science, traditional optics, ergonomics, precision machinery, modern electronics, mathematics, and software. It is widely used in medical applications. In ureteral soft endoscopic surgery, a soft ureteral endoscope can enter the body through the urethra to observe the diseased part, and can cooperate with surgical instruments to perform visual surgical treatment in the body. Clinical in-vivo examinations and visual surgical treatments require clear images of endoscopes to help doctors improve the accuracy of diagnosis. However, the existing endoscopes used for soft ureteral surgery are tube endoscopes. The human body was injured during the operation, but due to the difficulty of advancing the hose after the resistance, it was difficult for the hose to reach the surgical site accurately, and it could not accurately reflect the pathological condition of the diseased part, resulting in an unsuccessful surgical process, increasing the difficulty of the operation, and increasing the operation time and patient pain also increase the cost of surgery.

### SUMMARY

To overcome the above defects mentioned in the background of this invention, the invention disclosure provides an endoscope operating sheath for urological surgery, which can accurately reach the surgical site, and cooperate with surgical instruments and suck out broken stones and reduce intra-renal pressure by reaching to the surgical site through a visible hard sheath or a soft-hard sheath.

To achieve this object, provided is an endoscope operating sheath for urological surgery, comprising an endoscope handle and an endoscope operating sheath, wherein it further comprises a core rod disposed in the endoscope operating sheath and capable of sliding along an axial direction of the endoscope operating sheath; the core rod comprises a guide wire passage, a water injection passage, and an imaging passage which are sequentially connected to one another; the endoscope handle comprises a guide wire connector configured to guide a lead wire into the guide wire passage, a water injection connector (the water injection passage and the guide wire passage are interchangeable) configured to guide water into the water injection passage, and an endoscopy connector (the endoscopy can be cured in the core rod in advance) configured to guide an endoscope into the imaging passage.

The endoscope operating sheath can be a hard sheath or a soft-hard sheath.

When the endoscope operating sheath is a hard sheath, a rear end of the hard sheath is fixed in the endoscopy handle and a front end of the hard sheath is exposed out of the endoscope handle; the hard sheath comprises an axial straight hole, and the axial straight hole is in clearance fit with the core rod.

When the endoscope operating sheath is the endoscope operating sheath is a soft-hard sheath, it comprises a hard sheath whose rear end is fixed in the endoscope handle and front end exposed out of the endoscope handle and a soft sheath whose rear end is coaxial and integrated with the front end of the hard sheath; an inner diameter and an outer diameter of the soft sheath are the same as that of the hard sheath (the material of the hard sheath is same with/different from that of the soft sheath, and the hardness of the soft sheath can be adjusted by density of spring wires in the soft-hard sheath); the soft sheath and the hard sheath each comprise an axial straight hole along each axial directions of the soft sheath and the hard sheath, and the axial straight hole is in clearance fit with the core rod; a steering operation mechanism is provided on the endoscope handle, the steering operation mechanism is fixed to a steering wire of the soft sheath.

No matter the endoscope sheath is a soft-hard sheath or a hard sheath, the outer surface of the sheath is a hydrophilic coating; the inner surface is made of a hydrophilic material, and its role is to make the surface of the sheath lubricate so that the sheath is harmless or minimally damaged when entering into the human body. The inner and outer surface treatment method of the sheath is not limited to the method of the present invention.

Further, the front end of the soft sheath is fixed with a locking cap (it is not limited to metal material), the locking cap is coaxial with the soft sheath; one end of the steering wire is fixed on the locking cap, and the other end of the steering wire passes through the soft sheath and the hard sheath to fixedly connect to the steering operation mechanism.

More preferably, one side of the endoscope handle is fixed with a hand shank, the steering operation mechanism is disposed on the hand shank, and the hand shank is provided with a plurality of holding grooves which are corresponding to fingers. There is no handle on the endoscope handle of the hard sheath.

Further preferably, the handle may be fixed on the side of the handle sleeve, but is not limited to this fixing method.

Specially, a core rod card connector is fixed to and cooperated with a rear end of the endoscopy handle; the guide wire connector, the water injection connector and the endoscopy connector are disposed on the core rod card connector.

Specially, the endoscope handle comprises a handle sleeve, the handle sleeve is provided with an axial through hole therein, the axial through hole comprises a hard sheath straight hole, a handle core rod straight hole and a handle core rod taper hole; the hard sheath straight hole locates on the front end of the handle sleeve, the rear end of the hard sheath passes through the hard sheath straight hole and fixes inside the handle sleeve, the handle core rod straight hole communicates with a rear end of the hard sheath straight hole, the handle core rod taper hole communicates with the handle core rod straight hole and extends to the rear end of the handle sleeve; the rear end of the handle sleeve is provided with a core rod card connector.

Specially, the core rod card connector comprises a card connector casing, a front end of the card connector casing is provided with a core rod location counter bore configured to match with the rear end of the core rod, an inner end face of the core rod location counter bore is provided with a guide wire hole corresponding to the guide wire passage, a water injection hole corresponding to the water injection passage and a endoscopy hole corresponding to the imaging passage; the guide wire connector, the water injection connector and the endoscopy connector communicate with the guide wire hole, the water injection hole and the endoscopy hole respectively; the surfaces of two sides of the card connector casing each are fixed to a fastener via a fastener connection plate; two sides of the rear portion of the handle sleeve each are provided with a clasp locating slot, and the clasp locating slot matches with the fastener in shape.

Preferably, the diameters of the three passages of the guide wire passage, the water injection passage and the imaging passage are the same, and the inner diameters of guide wire connector, the water injection connector and the endoscope joint are the same. The front end of the image passage is provided with a shielding glass that does not affect the image effect.

More preferably, the guide wire passage, the water injection passage and the imaging passage of the core rod are independent from each other. Alternatively, the guide wire passage of the core rod shares one passage with the water injection passage of the core rod, and the imaging passage is an independent passage. The guide wire passage and the water injection passage are combined into one to form a larger instrument passage than the imaging passage; the instrument passage may be a round hole or an irregular curved hole, that is, the sheath core of the entire endoscope sheath has two passages with different aperture sizes and shapes.

Specially, the rear end of the handle sleeve is fixed with a clasp disc; the clasp disc is coaxial with the handle sleeve, a center of the clasp disc is provided with a clasp disc core rod via hole, the clasp disc core rod via hole communicates with the handle core rod taper hole and is coaxial with the handle core rod taper hole, a rear portion of the clasp disc is connected with a circumferential flange, and the clasp locating slot is disposed on the circumferential flange.

Preferably, two connection rods are connected to the circumferential flange, and one end of each of the two connection rods is provided with a string bag for accommodating broken stones.

More preferably, one side of the handle sleeve is fixed with a vacuum suction device interface, the vacuum suction device interface communicates with the handle core rod taper hole, the vacuum suction device interface connects with a vacuum suction device joint corresponding to a vacuum suction device, a water inlet of the vacuum suction device interface is located above a rear end of the hard sheath straight hole.

Preferably, another vacuum suction device interface or another water injection interface may be disposed on the other side corresponding to the vacuum suction device interface on one side of the handle sleeve.

More preferably, in order to effectively reduce the intra-renal pressure, a number of circular pressure-relief holes arranged in different ways may be opened around the head ends of the soft-hard sheath, the soft sheath of the hard sheath or the hard sheath.

Advantages of the endoscope operating sheath for urological surgery according to embodiments of the invention are summarized as follows. As a transitional guiding device, the core rod can be used in the urological surgery with existing endoscopes to guide the ureteroscope to reach the operative site accurately for providing a basis for later surgery made with soft mirrors. It is mainly used as a visible ureteral expansion sheath in renal pelvis, calculus stones and other related operations. Specifically, the core rod and the endoscope sheath are guided by the lead wire to the operative site. The ureter can be observed under the endoscope, and when the ureter is blocked, the water can be injected into the axial through hole to remove the obstacle. When the core rod and the endoscope operating sheath reach the operative site, the core rod is withdrawn, a ureteroscope is inserted in the endoscope operating sheath to perform flexible ureteroscopy. The main components of the endoscope operating sheath comprises: 1. a hard sheath having an operation handle, negative pressure suction interface (1-2 interfaces) or a flexible soft-hard sheath; 2. A core rod having an endoscope passage, a guide wire passage and a water injection passage; 3. a string bag for collecting the stones discharged in surgery. During the operation, the position of the ureteroscope can be adjusted by turning the steering operation mechanism, so that the soft and hard sheath can enter the renal pelvis and calyces and can be positioned. The ureteroscope, together with the operation instrument, breaks the stones in the renal pelvis and calyces. A vacuum suction device is connected to the suction passage of the endoscopy handle. When the broken stones are observed, the vacuum machine is started, and the front end of the ureteroscope exits from the soft sheath. The negative pressure in the soft sheath will suck the broken stones out of the body. The broken stones are placed in the string bag. After the operation, the broken stones in the string bag are treated. Based on the endoscope operating sheath for urological surgery comprising the core rod and the core rod card connector, the ureteroscope can move to the operative site accurately. The operation is easy and convenient, can reduce the amount of bleeding and the pain of the patients, improves the operation efficiency and success rate, reduces the operation time, and has good market application value and medical value. It is applicable but not limited to cardiovascular intervention, gynecology, thyroid and breast surgery, neurosurgery and orthopedics, and other related clinical medical fields.

### BRIEF DESCRIPTION OF THE DRAWINGS

FIG. **1** is a stereogram of an endoscope operating sheath for urological surgery comprising a hard sheath according to one embodiment of the invention;
FIG. **2** is a cooperative diagram of a hard sheath and a core rod card connector according to one embodiment of the invention;
FIG. **3** is a front view of an endoscope operating sheath for urological surgery comprising a hard sheath according to one embodiment of the invention;
FIG. **4** is a sectional view taken from line A-A in FIG. 3;
FIG. **5** is a side view of an endoscope operating sheath for urological surgery comprising a hard sheath according to one embodiment of the invention;
FIG. **6** is a sectional view taken from line B-B in FIG. **5**;
FIG. **7** is a stereogram of an endoscope operating sheath for urological surgery comprising a hard sheath and a soft sheath according to another embodiment of the invention;
FIG. **8** is a front view of an endoscope operating sheath for urological surgery comprising a hard sheath and a soft sheath according to another embodiment of the invention;
FIG. **9** is a sectional view taken from line C-C in FIG. **8**;
FIG. **10** is a side view of an endoscope operating sheath for urological surgery comprising a soft-hard sheath according to another embodiment of the invention;
FIG. **11** is a sectional view taken from line D-D in FIG. **10**;
FIG. **12** is a front view of a core rod card connector according to one embodiment of the invention;
FIG. **13** is a sectional view taken from arrow K in FIG. **12**;
FIG. **14** is a front view of a core rod according to one embodiment of the invention;
FIG. **15** is a top view of a core rod according to one embodiment of the invention;
FIG. **16** is a front view of an endoscope handle according to one embodiment of the invention;
FIG. **17** is a sectional view taken from line E-E in FIG. **16**; and
FIG. **18** is a schematic diagram of a clasp disc according to one embodiment of the invention.

In the drawings, the following reference numbers are used: **1.** Endoscope handle (**1.1.** Handle sleeve; **1.2.** Clasp disc; **1.3.** Hand shank); **2**. Endoscope operating sheath(**2.1.** Hard sheath; **2.2.** Soft sheath); 3. Core rod; **4.** Core rod card connector (**4.1.** Card connector casing; **4.2.** Card connector location counter bore); **5.** Guide wire passage; **6.** Water injection passage; **7.** Imaging passage; **8.** Guide wire connector; **9.** Water injection connector; **10.** Endoscope connector; **11.** Steering wire; **12.** Steering operation mechanism; **13.** Hard sheath straight hole; **14.** Handle core rod taper hole; **15.** Clasp locating slot; **16.** Guide wire hole; **17.** Water injection hole; **18.** Endoscope hole; **19.** Fastener; **20.** Locking cap; **21.** Clasp disc core rod via hole; **22.** Circumferential flange; **23.** Connection rod; **24.** String bag; **25.** Vacuum suction device interface; **26.** Holding groove; **27.** Fastener connection plate; **28.** Location blocks; **29.** Circumferential flange slot; **30.** Vacuum suction device joint; **31.** Handle seal plug; **32.** Handle core rod straight hole.

### DETAILED DESCRIPTION OF THE EMBODIMENTS

To further illustrate, embodiments detailing an endoscope operating sheath for urological surgery are described below.

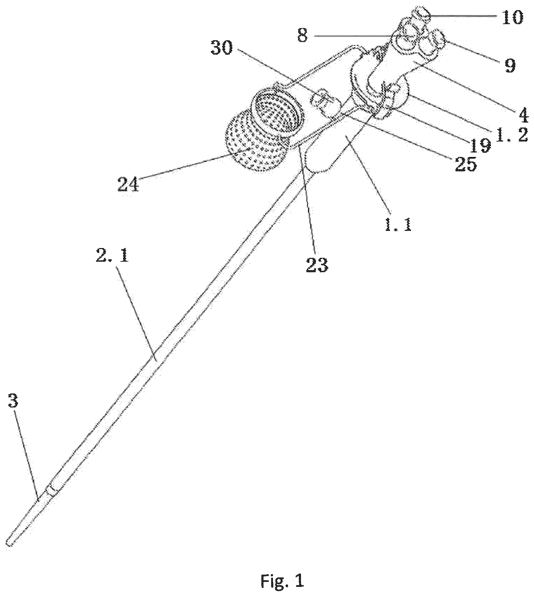

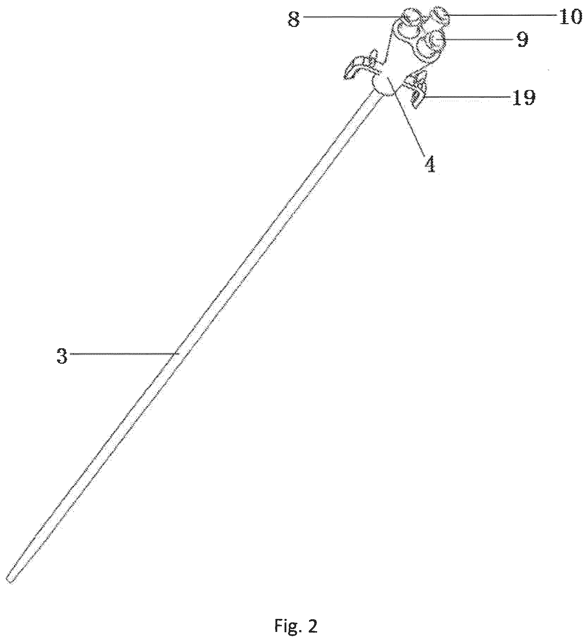

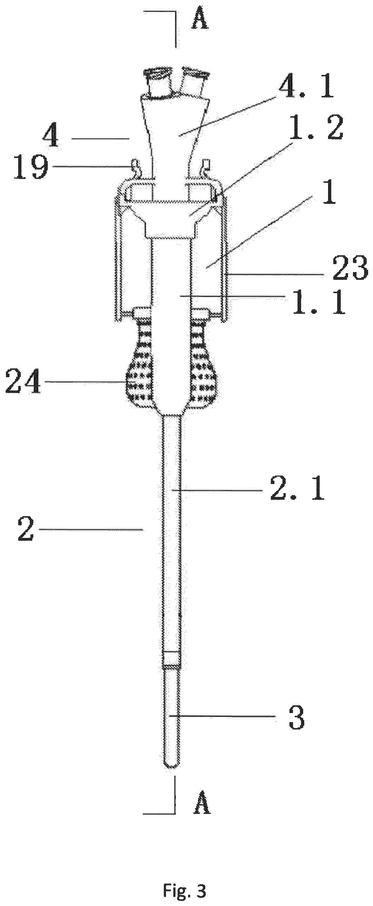

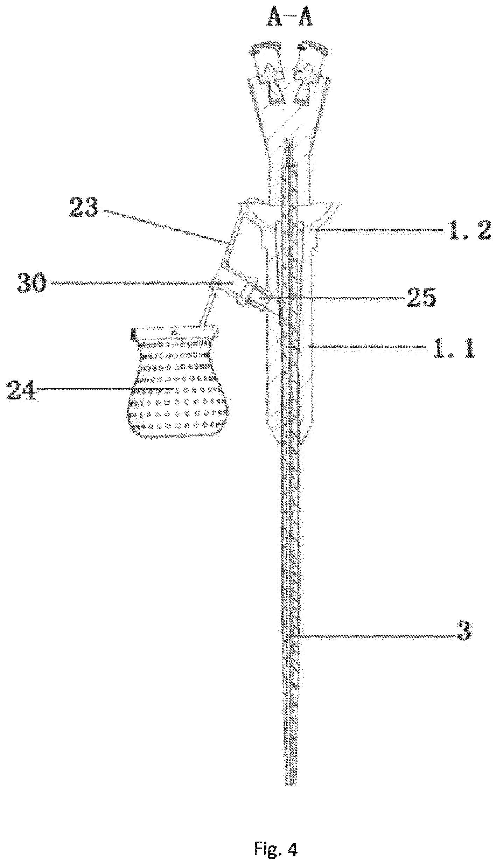

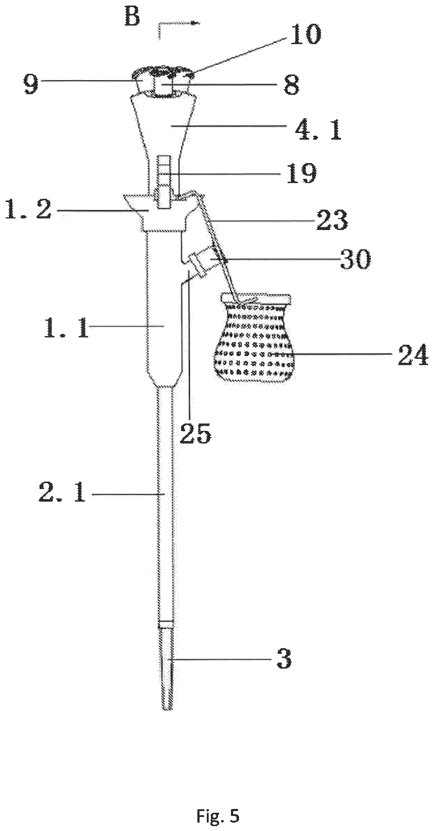

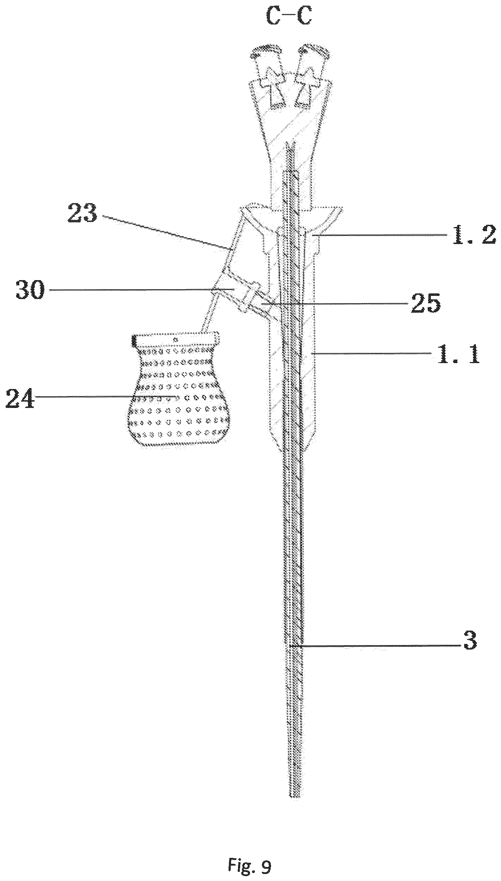

FIGS. **1-18** show an endoscope operating sheath for urological surgery for urological operations. The endoscope operating sheath for urological surgery comprises an endoscope handle **1**, an endoscope operating sheath **2** and a core rod **3** disposed in the endoscope operating sheath **2** and capable of sliding along an axial direction of the endoscope operating sheath **2.** The cord rod **3** is made of is medical polymer material (but not limited to polymer material), which has certain plasticity and can deform and bend, so that the cord rod can enter the human body and reach the pathological part conveniently.

The endoscope operating sheath **2** is a hard sheath **2.1** or a soft-hard sheath (comprising a hard sheath **2.1** and a soft sheath **2.2**).

As shown in FIGS. **1-6** and FIG. **17**, a rear end of the hard sheath **2.1** is fixed in the endoscope handle 1 and a front end thereof is exposed out of the endoscope handle **1.** The hard sheath **2.1** comprises an axial straight hole, and the core rod **3** is in clearance fit with the axial straight hole.

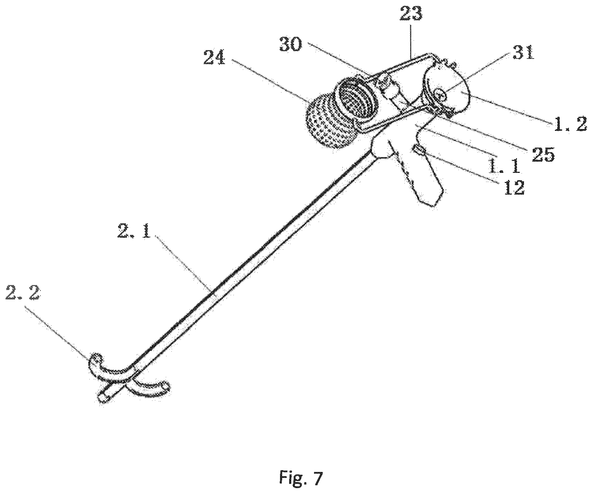

As shown in FIGS. 7-18, the soft-hard sheath comprises a hard sheath 2.1 and a soft sheath 2.2. A rear end of the hard sheath 2.1 is fixed in the endoscope handle 1 and a front end thereof is exposed out of the endoscope handle **1.** A rear end of the soft sheath **2.2** is coaxial and integrated with the front end of the hard sheath 2.1. An inner diameter and an outer diameter of the soft sheath **2.2** are the same as that of the hard sheath **2.1.** The soft sheath **2.2** and the hard sheath **2.1** each comprise an axial straight hole, and the core rod **3** is in clearance fit with the axial straight hole. The soft sheath **2.2** is provided with a steering wire **11**, and the endoscope handle **1** comprises a steering operation mechanism **12** on which the steering wire **11** is fixed.

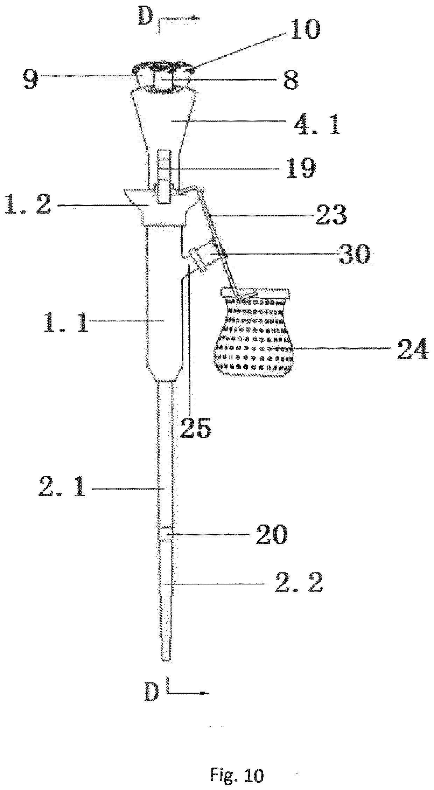

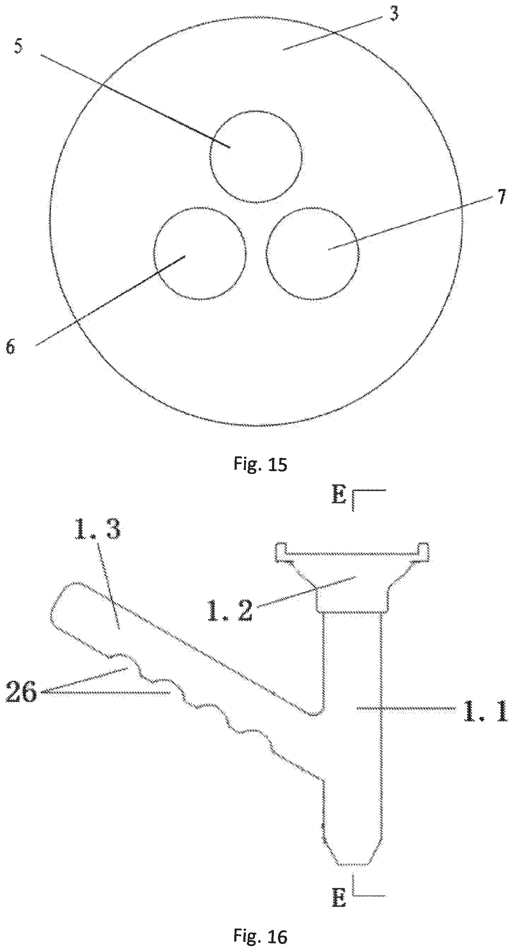

As shown in FIGS. 1-18, a rear end of the handle sleeve 1.1 is provided with a core rod card connector 4. The endoscope handle 1 comprises a handle sleeve 1.1, and an axial through hole is provided in the endoscope handle 1. As shown in FIGS. 16-17, the axial through hole of the handle sleeve 1.1 comprises a hard sheath straight hole 13, a handle core rod straight hole 32, and a handle core rod taper hole 14, the hard sheath straight hole 13 locates on the front end of the handle sleeve 1.1, the handle core rod straight hole 32 communicates with the rear end of the hard sheath straight hole 13, and the handle core rod taper hole 14 communicates with the rear end of the handle core rod straight hole 32 and extends to the rear end of the handle sleeve 1.1. The rear end of the handle sleeve **1.1** is provided with a clasp disc **1.2.** The clasp disc 1.2 is coaxial with the handle sleeve 1.1. One side of the handle sleeve **1.1** is provided with a hand shank **1.3** and a vacuum suction device interface 25. A vacuum suction device joint 30 corresponding to a vacuum suction device connects to the vacuum suction device interface 25. As shown in FIG.10, the vacuum suction device interface 25 communicates with the handle core rod taper hole 14 and a water inlet of the vacuum suction device interface 25 locates above the hard sheath 2.1 (so that the broken stone can enter the vacuum suction device interface 25 from the handle core rod taper hole 14 of the axial through hole of the core rod). When the suction passage is not in use, a plug can be used to block the vacuum suction device joint **30**, to avoid the internal pollution of the endoscope.

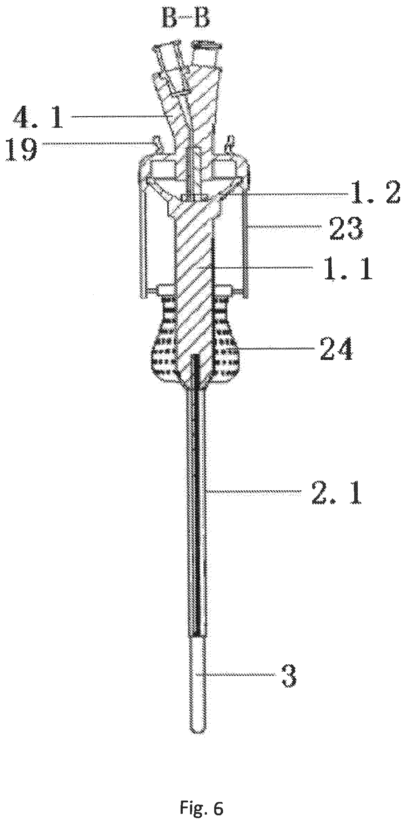

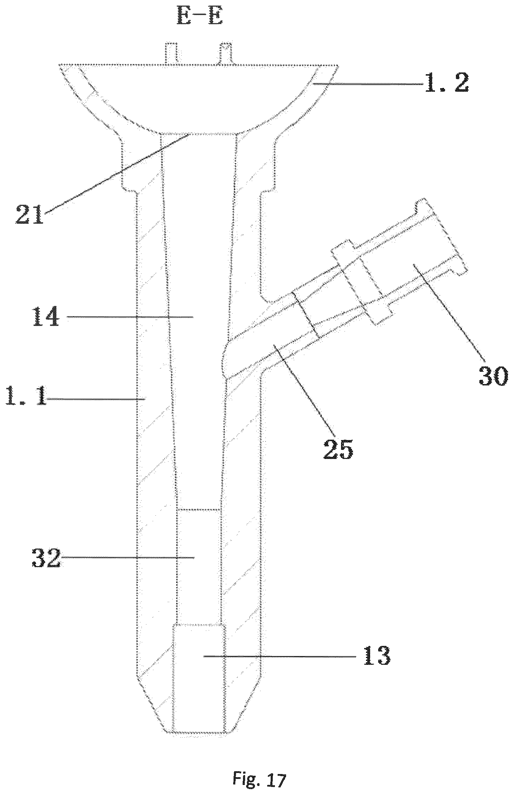

As shown in FIGS. 17-18, a clasp disc core rod via hole 21 is provided in the center of the clasp disc 1.2 and the clasp disc core rod via hole 21 communicates with and coaxial with the handle core rod taper hole 14 (as shown in FIG. 17, when no core rod is inserted, the clasp disc core rod via hole 21 is blocked by a handle seal plug 31, to avoid the internal pollution of the endoscope). A rear end of the clasp disc 1.2 connects with a circumferential flange 22. As shown in FIGS. 6-10, two connection rods 23 are connected to the circumferential flange 22, and one end of each of the two connection rods 23 is provided with a string bag 24 for accommodating broken stones. The handle core rod taper hole 14 is a taper hole of the core rod 3 and a taper hole of surgery soft endoscope. The endoscope enters the endoscope operating sheath 2 via an endoscopy connector 10.

As shown in FIGS. 7-11, the endoscope operating sheath **2** comprises a hard sheath 2.1 whose rear end is fixed in the hard sheath straight hole 13 of the endoscope handle 1 and front end exposed out of the endoscope handle 1 and a soft sheath 2.2 whose rear end is coaxial and integrated with the front end of the hard sheath 2.1. The soft sheath 2.2 and the hard sheath 2.1 each comprise an axial straight hole along the axial directions of the soft sheath 2.2 or the hard sheath 2.1.The axial straight hole is in clearance fit with the core rod 3. The front end of the soft sheath 2.2 is fixed with a locking cap 20, and the locking cap 20 is coaxial with the soft sheath 2.2. One end of the steering wire 11 is fixed on the locking cap 20 and the other end of the steering wire 11 passes through the soft sheath 2.2 and the hard sheath 2.1 to fixedly connect to the steering operation mechanism 12. The steering operation mechanism 12 is disposed on the hand shank 1.3. The hand shank 1.3 comprises a plurality of holding grooves 26 corresponding to fingers.

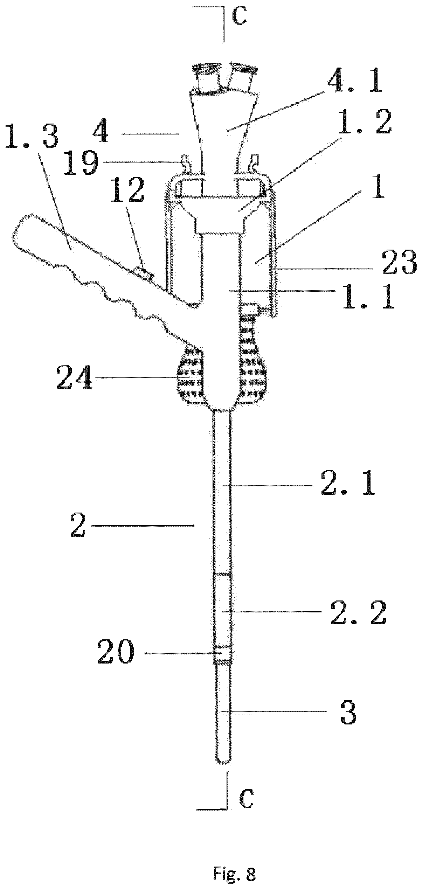

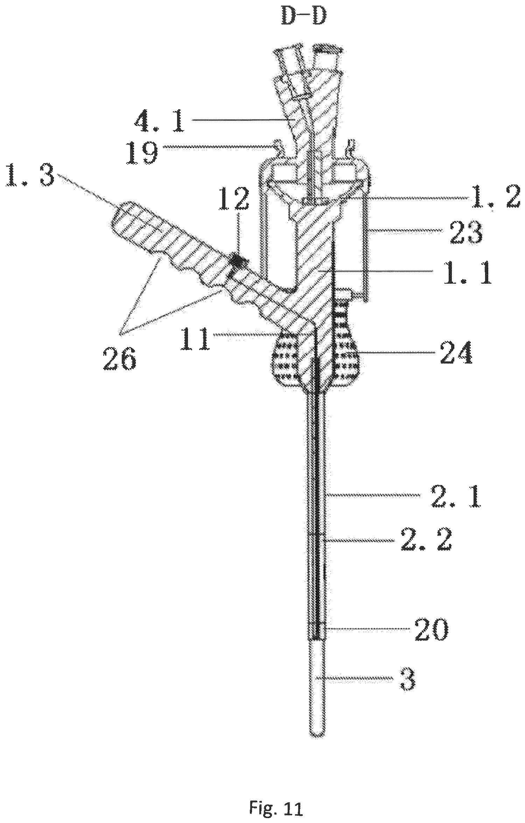

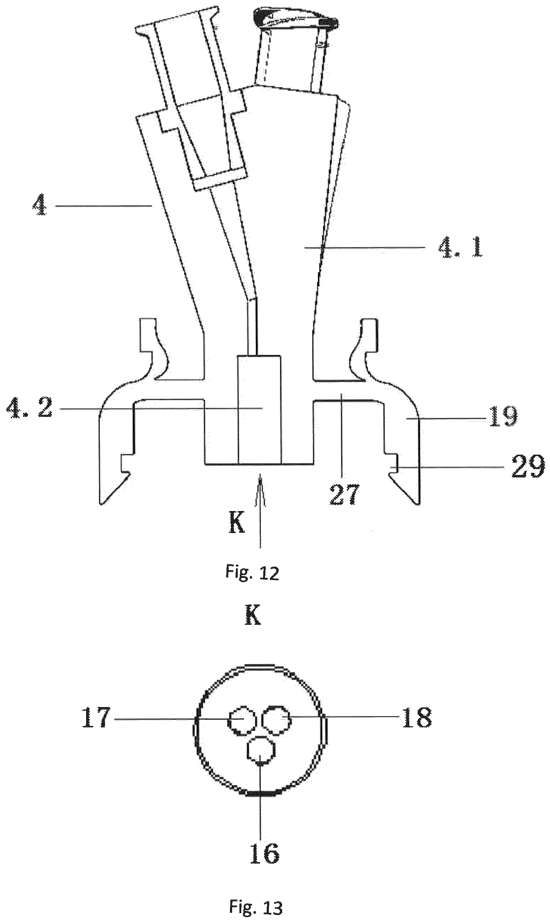

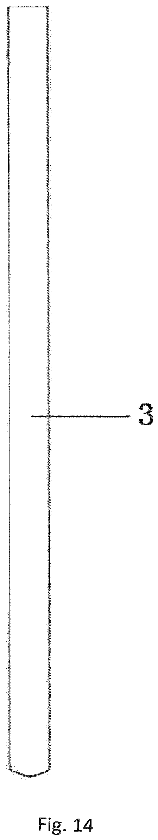

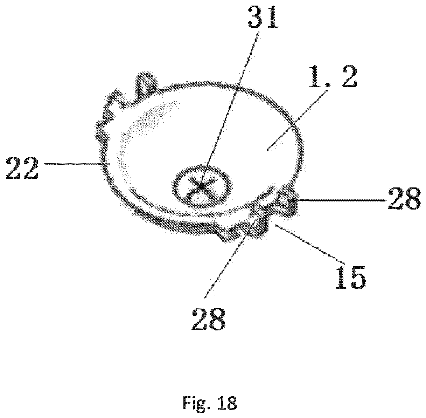

As shown in FIGS. 14-15, the core rod 3 comprises a guide wire passage 5, a water injection passage 6, and an imaging passage 7 which are sequentially connected to one another. As shown in FIGS. 8-12, the core rod card connector 4 comprises a card connector casing 4.1. A front end of the card connector casing 4.1 is provided with a core rod location counter bore 4.2 configured to receive the core rod 3. As shown in FIG. 7, the core rod location counter bore 4.2 comprises a guide wire hole 16 communicating with the guide wire passage 5, a water injection hole 17 communicating with the water injection passage 6, and an endoscope hole 18 communicating with the imaging passage 7. The guide wire connector 8, the water injection connector 9 and the endoscopy connector 10 communicate with the guide wire hole 16, the water injection hole 17 and the endoscope hole 18 respectively. The guide wire passage 5, the water injection passage 6 and the imaging passage 7 are all disposed in the core rod 3 and all have the same bore diameters. The guide wire connector 8, the water injection connector 9 and the endoscopy connector 10 all have the same size, and the front end of the imaging passage 7 is provided with glass to avoid the contamination of the endoscope entering the human body. The guide wire connector 8 and the water injection connector 9 can be replaced by one another, which improves the use efficiency of the endoscope during the operation and reduces the operation time. As shown in FIG. 11, two sides of the card connector casing 4.1 each are provided with a fastener 19 via a fastener connection plate 27. As shown in FIGS. 12 and 18, the circumferential flange 22 is provided with a clasp locating slot 15. The clasp locating slot 15 is consisted of four location blocks 28 and circumferential flanges 22. The gap between two location blocks 28 constitutes the location slot 15. After the fastener 19 is fixed on the flange 22 and a circumferential flange slot 29 of the fastener 19 engages with the circumferential flanges 22, the fastener 19 can be located on the circumferential flanges 22. The core rod 3 is first positioned in the core rod card connector 4, and then the endoscope operating sheath 2 is installed along the lead wire. When the core rod 3 and the endoscope operating sheath 2 reach the operating position, the core rod 3 and the core rod card connector 4 can be separated from the endoscope handle 1, thus achieving the detachment of the core rod 3. The arrangement of the core rod card connector 4 facilitates the positioning and detachment of the core rod 3, with the following advantages: in the process of installing the endoscope operating sheath, the ureter can be observed in real time, if being clogged, the obstacles can be removed instantly by water injection. Through the real-time observation, the position of the endoscope operating sheath can be accurately determined. After the endoscope operating sheath is in place, the core rod 3 is extracted, and the ureteroscopy is performed with an endoscope. The operation is performed under the endoscope, and the endoscope is moved forward in the hard and soft endoscope operating sheath to the renal pelvis and calyces. A suction machine is connected to the suction channel of the handle sleeve. The user grasps the endoscopy handle, bends the soft endoscope operating sheath to move the endoscope to the calyces. After the broken stones are found, the suction machine is started to suck out of the stones.

The use method of the endoscope operating sheath for urological surgery is described as follows. The endoscope operating sheath 2 and the core rod 3 are combined. The core rod card connector 4 and the endoscope handle 1 are combined (or the core rod card connector 4 and the endoscope handle 1 are not combined; one person holds the core rod card connector 4 for water injection operation, the other holds the endoscope handle 1 to make the endoscope operating sheath 2 enter the human body and control the direction of the endoscope operating sheath; the two persons operate the endoscope handle 1 and the core rod card connector 4 separately, thus reducing the difficulty and improving the operation efficiency). The endoscope is inserted into the imaging passage 7 of the core rod 3 (optionally, the endoscope can be fixed in the imaging passage 7 in advance), and the lead wire is inserted into the guide wire passage 5. The core rod 3 and the endoscope operating sheath 2 are guided by the lead wire to the operative site. The ureter can be observed under the endoscope, and when the obstacle occurs, the water can be injected into the water injection passage 6 to remove the obstacle. When the core rod 3 and the endoscope operating sheath 2 reach the operative site, the core rod 3 is extracted, a ureteroscope is inserted in the endoscope operating sheath 2 to perform flexible ureteroscopy. During the operation, the position of the ureteroscope can be adjusted by turning the Steering operation mechanism 12, so that the soft and hard endoscope operating sheath can enter the renal pelvis and calyces and can be positioned. The ureteroscope, together with the operation instrument, breaks the stones in the renal pelvis and calyces. A vacuum machine is connected to the suction channel of the endoscopy handle. When the broken stones are observed, the vacuum machine is started, and the front end of the ureteroscope exits from the soft sheath 2.2. The negative pressure in the soft sheath 2.2 will suck the broken stones out of the body. The broken stones are placed in the string bag 24. After the operation, the broken stones in the string bag 24 are treated. Based on the endoscope operating sheath for urological surgery comprising the core rod 3 and the core rod card connector 4 of the invention, the ureteroscope can move to the operative site accurately. The operation is easy and convenient, can reduce the amount of bleeding and the pain of the patients, and improves the operation efficiency and success rate.

It will be obvious to those skilled in the art that changes and modifications may be made, and therefore, the aim in the appended claims is to cover all such changes and modifications.

## Claims

1. An endoscope operating sheath for urological surgery, comprising an endoscope handle (1) and an endoscope operating sheath (2), wherein it further comprises a core rod (3) disposed in the endoscope operating sheath (2) and capable of sliding along an axial direction of the endoscope operating sheath (2); the core rod (3) comprises a guide wire passage (5), a water injection passage (6), and an imaging passage (7) which are sequentially connected to one another; the endoscope handle (1) comprises a guide wire connector (8) configured to guide a lead wire into the guide wire passage (5), a water injection connector (9) configured to guide water into the water injection passage (6), and an endoscopy connector (10) configured to guide an endoscope into the imaging passage (7); and a rear end of the endoscope operating sheath (2) is fixed in a front end of the endoscope handle (1) .

2. The endoscope operating sheath for urological surgery of claim 1, wherein the endoscope operating sheath (2) is a hard sheath (2.1); a rear end of the hard sheath (2.1) is fixed in the endoscope handle (1), a front end of the hard sheath (2.1) is exposed out of the endoscope handle (1); an axial straight hole is provided on the hard sheath (2.1) along an axial direction of the hard sheath (2.1), the axial straight hole is in clearance fit with the core rod (3).

3. The endoscope operating sheath for urological surgery of claim 1, wherein the endoscope operating sheath (2) is soft-hard sheath, it comprises a hard sheath (2.1) whose rear end is fixed in the endoscope handle (1) and front end exposed out of the endoscope handle(1) and a soft sheath (2.2) whose rear end is coaxial and integrated with the front end of the hard sheath (2.1); an inner diameter and an outer diameter of the soft sheath (2.2) are the same as that of the hard sheath (2.1); the soft sheath (2.2) and the hard sheath (2.1) each comprise an axial straight hole along each axial direction of the soft sheath (2.2) and the hard sheath (2.1), and the axial straight hole is in clearance fit with the core rod (3); a steering operation mechanism (12) is provided on the endoscope handle (1), the steering operation mechanism (12) is fixed to a steering wire (11) of the soft sheath (2.2).

4. The endoscope operating sheath for urological surgery of claim 3, wherein the front end of the soft sheath (2.2) is fixed with a locking cap (20), the locking cap (20) is coaxial with the soft sheath (2.2); one end of the steering wire (11) is fixed on the locking cap (20), and the other end of the steering wire (11) passes through the soft sheath (2.2) and the hard sheath (2.1) to fixedly connect to the steering operation mechanism (12).

5. The endoscope operating sheath for urological surgery of claim 4, wherein one side of the endoscope handle (1) is fixed with a hand shank (1.3), a steering operation mechanism (12) is disposed on the hand shank (1.3), and the hand shank (1.3) is provided with a plurality of holding grooves (26) which are corresponding to fingers.

6. The endoscope operating sheath for urological surgery of claim 2 or claim 3, wherein the endoscope handle (1) comprises a handle sleeve (1.1), the handle sleeve (1.1) is provided with an axial through hole therein, the axial through hole comprises a hard sheath straight hole (13), a handle core rod straight hole (32) and a handle core rod taper hole (14); the hard sheath straight hole (13) locates on the front end of the handle sleeve (1.1), the rear end of the hard sheath (2.1) passes through the hard sheath straight hole (13) and fixes in the handle sleeve (1.1), the handle core rod straight hole (32) communicates with a rear end of the hard sheath straight hole (13), the handle core rod taper hole (14) communicates with the handle core rod straight hole (32) and extends to the rear end of the handle sleeve (1.1); the rear end of the handle sleeve (1.1) is provided with a core rod card connector (4), and the guide wire connector (8), the water injection connector (9) and the endoscopy connector (10) are disposed on the core rod card connector (4).

7. The endoscope operating sheath for urological surgery of claim 6, wherein the core rod card connector (4) comprises a card connector casing (4.1); a front end of the card connector casing (4.1) is provided with a core rod location counter bore (4.2) configured to match with the rear end of the core rod (3), an inner end face of the core rod location counter bore (4.2) is provided with a guide wire hole (16) corresponding to the guide wire passage (5), a water injection hole (17) corresponding to the water injection passage (6) and a endoscopy hole (18) corresponding to the imaging passage (7); the guide wire connector (8), the water injection connector (9) and the endoscopy connector (10) communicate with the guide wire hole (16), the water injection hole (17) and the endoscopy hole (18) respectively; the surfaces of two sides of the card connector casing (4.1) each are fixed to a fastener (19) via a fastener connection plate (27); two sides of the rear portion of the handle sleeve (1.1) each are provided with a clasp locating slot (15), and the clasp locating slot (15) matches with the fastener (19) in shape.

8. The endoscope operating sheath for urological surgery of claim 7, wherein the rear end of the handle sleeve (1.1) is fixed with a clasp disc (1.2); the clasp disc (1.2) is coaxial with the handle sleeve (1.1), a center of the clasp disc (1.2) is provided with a clasp disc core rod via hole (21), the clasp disc core rod via hole (21) communicates with the handle core rod taper hole (14) and is coaxial with the handle core rod taper hole (14), a rear portion of the clasp disc (1.2) is connected with a circumferential flange (22), and the clasp locating slot (15) is disposed on the circumferential flange (22).

9. The endoscope operating sheath for urological surgery of claim 8, wherein two connection rods (23) are connected to the circumferential flange (22), and one end of each of the two connection rods (23) is provided with a string bag (24) for accommodating broken stones.

10. The endoscope operating sheath for urological surgery of claim 6, wherein one side of the handle sleeve (1.1) is fixed with a vacuum suction device interface (25), the vacuum suction device interface (25) communicates with the handle core rod taper hole (14), the vacuum suction device interface (25) connects with a vacuum suction device joint (30) corresponding to a vacuum suction device, a water inlet of the vacuum suction device interface (25) is located above a rear end of the hard sheath straight hole (13).

11. The endoscope operating sheath for urological surgery of claim 1, wherein the guide wire passage (5) of the core rod (3) shares one passage with the water injection passage (6) of the core rod (3), and the imaging passage (7) is an independent passage.

12. The endoscope operating sheath for urological surgery of claim 1, wherein the guide wire passage (5), the water injection passage (6) and the imaging passage (7) of the core rod (3) are independent from each other.
